# EUROPEAN PATENT APPLICATION

(11) **EP 2 910 194 A1**
(43) Date of publication of application: **26.08.2015**
(21) Application number: 14156155.5
(22) Date of filing: 21.02.2014
(51) Int. Cl.: A61B 17/00, A61B 17/29, A61B 19/00

(54) **Surgical instrument**

(71) Applicant: 3DIntegrated ApS, 8541 Skodstrup (DK)
(72) Inventor: Møller Hansen, Steen, 8541 Skødstrup (DK)
(74) Representative: Hegner, Anette

(57) **Abstract**

The invention regards a surgical instrument for use in minimal invasive surgery wherein a surgeon performs a surgical procedure within a body cavity with indirect vision of a surgical field, the surgical instrument comprises, a handle portion for manipulation of the instrument, and a body portion extending from the handle portion and comprising a surgical tool, wherein the body portion is adapted to be inserted through an incision in a body into the body cavity, characterised in that the body portion further comprises a pattern generating member which, when the body part is inserted into the body cavity, projects a light pattern on an area of the surgical field such that the contours of the surgical field and the position of the instrument can be deduced by the surgeon based on indirect vision of the light pattern.

## Description

### Field of invention

Surgical instrument for use in a minimal invasive surgery

### Background of the Invention

Minimal invasive surgery has been used increasingly in the last years due to the benefits compared to conventional open surgery as it reduces the trauma to the patient tissue, leaves smaller scars, minimizes post-surgical pain and enables a faster recovery of the patient.

For example, in laparoscopic surgery (a form of minimal invasive surgery) the surgeon accesses a body cavity, such as the abdominal or pelvic cavity, through a series of small incisions. A laparoscope is inserted through an incision, and conventionally connected to a monitor, enabling the surgeon to see the inside of the abdominal or pelvic cavity. In order to perform the surgery, surgical instruments are inserted through incisions. In addition, the body cavity around the surgical site is inflated with e.g. carbon dioxide in order to create an air space within the cavity for the surgeon to view the surgical site and move the laparoscopic instruments.

In conventional open surgery the surgeon can use the normal visual-motor relations, wherein the motor control is based on visual perception, such that a desired movement of a surgical instrument can be performed on basis of vision. In other words, during conventional open surgery the normal link between the visual perception and the motor system is conserved. However, when performing minimal invasive surgery the surgeon has an indirect vision of the surgical field which results in dissociation of the visual perception and the motor system of the surgeon. Consequently, the surgeon needs to acquire new skills in order to correctly connect his or hers visual perception and motor system (hand-eye coordination) during minimal invasive surgery.

Visual perception is the ability to interpret the surrounding environment by processing information obtained by use of the eyes, in the present case the surrounding environment can be the inside of a body cavity,such as the abdominal or pelvic cavity. The motor system of a person is the complex system which, among other things, controls voluntary movements, enabling a surgeon to move body parts, such as a hand and fingers, to control the movement of a surgical instrument inside a body cavity.

Further, the remote vision of the surgical field is normally displayed on a monitor in two dimensions whereas the surgical instrument is manipulated in three dimensions; this results in a poor spatial and depth perception which makes it even harder for a surgeon to acquire the new abilities for connecting visual perception of the remote vision (in two-dimensions) and motor system moving the surgical tools (in three-dimensions).

In addition, if the surgical tools are controlled via a surgical robot the normal three dimensional motor behaviors of the surgeon are processed and changed by the robot, which makes it more difficult for the surgeon to correctly connect his or hers visual perception and motor system during minimal invasive surgery.

Training in minimal invasive surgery is normally performed after basic surgical training and is based on apprenticeship where the skills are obtained in direct clinical surgery supervised by an experienced surgeon. This training method poses considerable risk to the patient and requires a substantially amount of time from the experienced surgeon. Therefore, the use of simulators, for example laparoscopic simulators, is preferred in order for an inexperienced surgeon to learn the basic skills before starting to do clinical surgery. Among the most important skills needed to master is; the ability to transform the information received by indirect vision into a three dimensional understanding.

### Summary of the invention

Considering the problems mentioned above it is an object of the present invention to provide a surgical instrument for use in minimal invasive surgery which enhances the surgeon's visual perception such that the surgeon is able to connect his or hers visual perception and motor system during minimal invasive surgery whereby an intended movement of the surgical instrument can be performed on basis of remote vision.

The object can be achieved by means of a surgical instrument for use in minimal invasive surgery wherein a surgeon performs a surgical procedure within a body cavity with indirect vision of a surgical field, the surgical instrument comprises, a handle portion for manipulation of the instrument, and a body portion extending from the handle portion and comprising a surgical tool, wherein the body portion is adapted to be inserted through an incision in a body into the body cavity,
characterised in that the body portion further comprises a pattern generating member which, when the body part is inserted into the body cavity, projects a light pattern on an area of the surgical field such that the contours of the surgical field and the position of the instrument can be deduced by the surgeon based on indirect vision of the light pattern.

Thus, it is possible for the surgeon to use the light pattern as a reference in order to connect the remote vision with the movement of the surgical tools. The light pattern can be interpreted as a monocular reference which enables the surgeon to determine the position of the surgical instrument and the contours of the surgical field. Consequently, the present invention enables the surgeon to overcome the difficulties in connecting his or hers visual perception and motor system during minimal invasive surgery.

The indirect vision of the surgical field can be obtained through an endoscope inserted through an incision in the body. The endoscope can be connected to a monitor displaying the surgical field in a two dimensions image.

Light pattern can be a grid or a plurality of light dots that generate the pattern. It can also comprise one or more cones of light that forms a geometric shape, such as a square. The surgeon can then use the curves defined by the edges of the geometric shape to determine the position of the surgical instrument and the contours of the surgical field.

The handle portion can comprise an actual handle for the surgeon to seize and thereby control the surgical instrument directly. In another embodiment, the handle portion is controlled by use of an actuator connected to a control mechanism, for example a surgical robot, such that the surgeon can control the surgical instrument indirectly.

The surgical instrument mentioned above can both be used in training surgeons in minimal invasive surgery and during the actual surgical procedure. When used during training it will reduce the training time before a minimal invasive surgeon is sufficiently skilled to perform live surgery. When used during the actual surgical procedure it will help the surgeon, hereby minimising the risk of mistakes and secure a smoother surgical procedure.

Minimal invasive surgery can be laparoscopic surgery or a minimal invasive procedure in the thorax or abdomen.

In an embodiment the pattern generating member is detachably attached to the body portion. The surgeon can then remove the pattern generating member if needed. In addition, the pattern generating member can be attached to an existing surgical instrument in order to obtain an embodiment of the present invention.

In an embodiment, the light pattern is a grid. Thus, the light pattern is a light grid which is projected on an area of the surgical field. The changes in the grid lines can be used to deduce the contours of the body cavity and the changes in the angle between crossing grid lines when the surgical instrument can be used to determine the orientation of the surgical instrument.

Preferably, the light pattern comprises a plurality of light dots. When the surgical instrument is moved, the position and /or the distance between the dots will change which enhances the surgeon's ability even further to deduce the position of the surgical instrument and the contours area of the surgical field.

Advantageously, the light pattern is generated by at least one laser and/or LED. Lasers and LED's (light emitting diodes) are advantageous as they can generate light patterns that are well defined and it is possible to choose the wavelength, and thus colour, such that the pattern is enhanced in the remove vision for example such that the light pattern is clearly visible and enhanced on the monitor.

In an embodiment, the pattern generating member projects the light pattern on an area in front of the body portion covering 90 degree to the sides, preferably 60 degree. The light pattern can be projected in spherical originating in a centre where the pattern generating member is situated. In front of the body portion, is to be understood as along the longitudinal axle of the body portion and away from the handle portion, preferably it is in front of the surgical tool.

In an embodiment, the projected light pattern is adapted to be able to change form, shape, position and/or colour. The surgeon can then ensure that the light pattern has the optimal form and/or extend and/or position on the surgical area for further enhancing the surgeon's ability to coordinate movement of the surgical instrument based on the indirect vision. In an example the colour of the light pattern is chosen such that specific objects in the surgical field appear clearer and/or with a larger contrast in relation to other objects in the surgical filed. This can be used to highlight an object in the surgical field, for example an organ which is the target for the surgical procedure.

In an embodiment, data on position and/or changes in the light pattern is obtained and a system is adapted to receive the data and determine the position of the surgical tool, preferably the information of the position of the surgical tool is presented to an operator. This can for example, be information of the distance between the surgical tool and the surface of the surgical field. This distance can be presented a monitor used for remote vision as a distance in mm. The distance can be given for any distance between the surgical tool and another object in the body cavity.

In an embodiment, data of the wavelength of the reflected light of the light pattern is obtained and a system is adapted to receive the data and determine properties of tissue in the surgical field. The information of the light emitted by the pattern generating member can be very well defined and by analysing the reflected light, the light pattern makes it possible to determine properties of the tissue. This can for example be the oxygen level in the tissue and changes thereof, and the type of tissue. For example the reflected light can be used to determine what kind of organ the tissue is part of, which indicates to the surgeon what organs are which and thereby assisting the surgeon to the area of interest.

In an embodiment, at least one light source and at least one optical waveguide is connected to the pattern generating member, wherein the at least one optical waveguide guides the light generated by the light source to where the light is projected. The optical waveguide can be a fibre optic cable. Preferably, the light source is not intended to be inserted into the body cavity and the at least one optical waveguide is adapted to guide the light through the incision in the body. Thus, pattern generating member that is inserted in to the body cavity can be made relatively small, as the light can be generated outside the body cavity and guided via optical waveguides to the body cavity where it can be projected onto the area of the surgical field. Advantageously, the light source can be detached from the at least one optical waveguide. This simplifies the sterilisation process of the surgical instrument as the light source does not need to be sterilised because it does not enter the body cavity. In an example, there is one light source that is either connected to one optical waveguide or a plurality of optical waveguides. In another example, there are more than one light source which made by one or more optical waveguides.

The invention further regards a method for training the handling of a surgical instrument having a handle portion for manipulation of the instrument, and a body portion extending from the handle portion and comprising a surgical tool, wherein the body portion further comprises a pattern generating member which projects a light pattern, the method comprises the steps; provide a cavity, provide a system for indirect vision of an area of the cavity, insert the surgical tool and at least a section of the body portion into the cavity, project the light pattern on a wall of the cavity visible by the system for indirect vision, move the instrument and observe, via the system for indirect vision, the changes in the light pattern.

Thus, it is possible to train the handling of a surgical instrument based on remote vision of a section of the surgical instrument inside a cavity. Using this method for training, the handling of the surgical instrument, will enhance the ability to transform the information revived by indirect vision into a spatial understanding of the position of the instrument and the internal contours of the cavity. Hence, it will train the ability to correctly connect the visual perception and motor system (hand-eye coordination) when observing via remote vision.

In an embodiment, the method is adapted such that data of the position and/or changes in the light pattern is obtained and forwarded to a system, and wherein the system is adapted to determine the abilities of an operator of the surgical instruments based on an evaluation of the data of the position and/or changes in the light pattern. The operator, such as the surgeon in training, will move the surgical instrument during training in order to perform the task given. If the position and/or movement of the light pattern is recorded by a system (e.g. a computer) connected to the remove vision, (e.g. a laparoscope) the operators ability to handle the surgical instrument can be determined. For example, if the light pattern has repetitive changes it can indicate that the operator makes the same movements of the surgical instruments several times and thus have difficulty doing a specific task, for example aligning the instrument for grapping a specific item in the surgical field. Thus, the computer can so to speak, give a mark of the performance of the surgeon in a training session based on data of the position and/or changes in the light pattern. It is to be understood that the abovementioned feature also can be used during live surgery for evaluating the surgeon's capabilities during the surgical procedure.

In addition the invention regards a minimal invasive surgery using a surgical instrument or method as described herein is used.

### Description of the drawings

The invention will in the following be described in greater detail with reference to the accompanying drawings:
- Fig. 1: a schematic view of an embodiment of a surgical instrument.
- Fig. 2: a schematic view of a body portion of an embodiment of a surgical instrument.
- Fig. 3: a schematic view of a body portion of an embodiment of a surgical instrument.
- Fig. 4: a schematic view of a light pattern generated by an embodiment of a surgical instrument.
- Fig. 5: a schematic view of a light pattern generated by an embodiment of a surgical instrument.
- Fig. 6: a schematic view of a projected light pattern by an embodiment of a surgical instrument.

### Detailed description of the invention

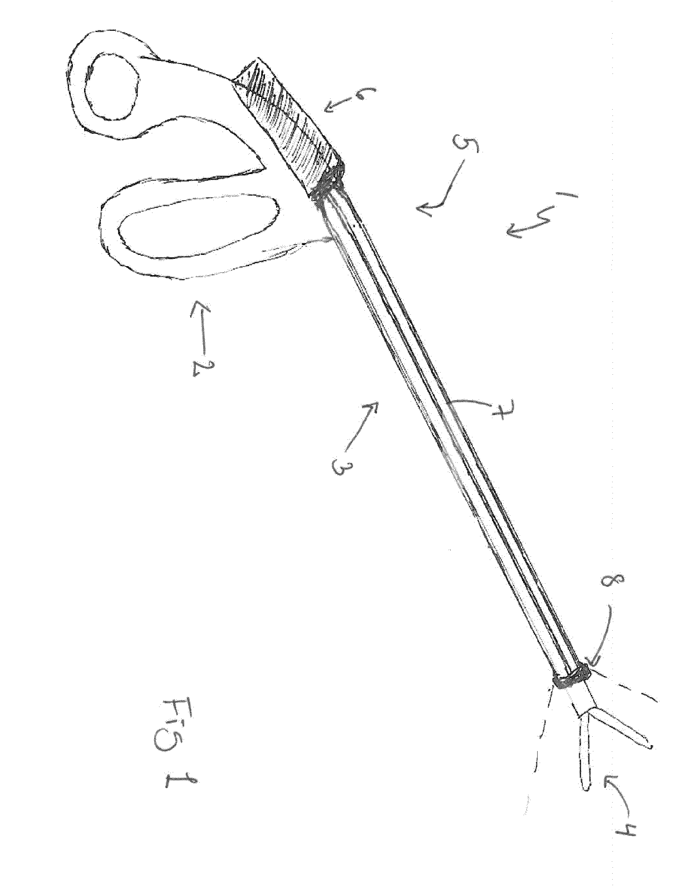

The figures are only schematic and are not drawn to scale. Fig. 1 discloses a surgical instrument 1, in the present case a laparoscopic instrument, with a handle portion 2 and a body portion 3 with a surgical tool 4 in the present case forceps, but can in another embodiment be any surgical tool, for example scissors. The surgeon holds the handle portion 2 and can in this way control the surgical instrument and by pressing the handle the forceps can be controlled.

The surgical instrument 1 further comprises a pattern generating member 5 comprising a light source 6 and optical fibres 7 which guide the light to a light emitting portion 8 where optical fibres 7 ends and the light is projected. The light sources 6 is placed on the handle portion 2 of the surgical instrument 1 and are thus not inserted into the body cavity during surgery. The light emitting portion 8 has a spherical geometry, hereby emitting a light pattern in a spherically whereby the light pattern forms a light pattern resembling the light pattern generated by a mirror ball. It is to be understood that the mirror ball resembling pattern is preferably only a section of normal mirror ball pattern, e.g. not a 360 degree pattern but preferably only a 90 degree section of the pattern. This light pattern makes it possible for the user of the surgical instrument 1 to deduce the position and movements of the surgical instrument 1 ad thus the surgical tool, relative to the surface where the light pattern is projected.

The light generated in the light source 6 may be generated by use of one or more LED's and/or lasers or a combination thereof.

A section of the body portion 3 of the surgical instrument 1 shown in fig. 1 is adapted to be inserted into a body cavity of a patient through small incisions. The surgeon then seizes the handle portion 2 with his or hers hands in order to perform the surgical procedure. An endoscope, e.g. a laparoscope, is also inserted into body cavity in order to let the surgeon see the surgical field where the surgical procedure is to be performed. The endoscope can comprise a video camera such that the surgeon can see the surgical field and the surgical instruments during the procedure on a monitor.The light emitting portion 8 of the pattern generating member 5 is inserted into the body cavity together with the surgical tool 4. The light emitting portion 8 will then project a light pattern on the surgical field. The light pattern will aid the surgeon to interpret the position and the contours of the surgical field. The light pattern aids the surgeon to connect his visual perception of the surgical area which is based on observing the monitor (two-dimensions) and his or hers motor system which is used to control the surgical tools which are moved within the body cavity (in three-dimensions).

The pattern generating member 5 or parts thereof can be made as a clip-on device or permanently attached to the body portion 3. In an embodiment the entire pattern generating member 5 is positioned on the body portion 3 and is adapted to be inserted into the body cavity.

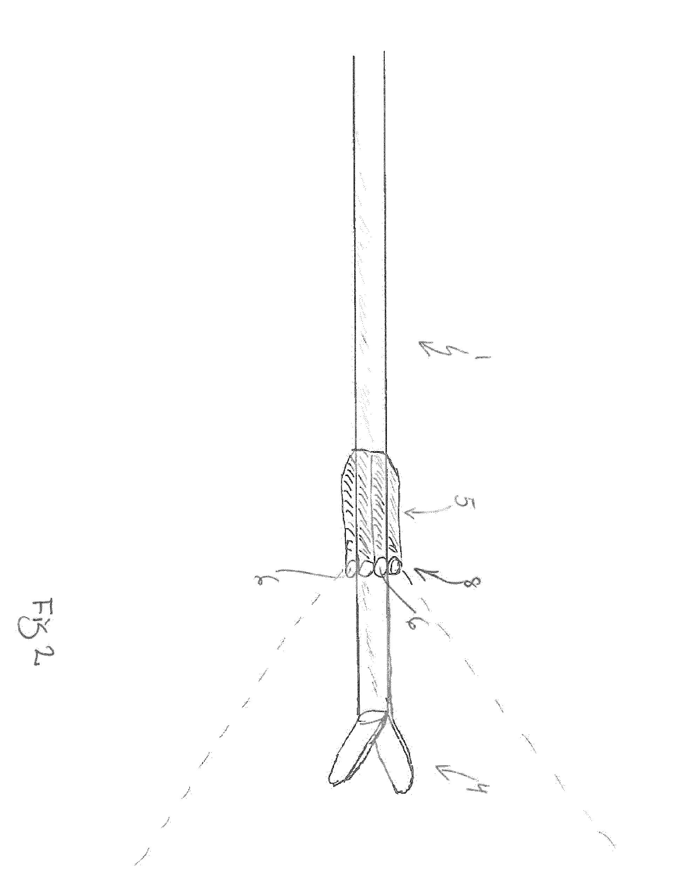

Fig. 2 shows a body portion 3 of a surgical instrument 1. The body portion 3 has a surgical tool 4 in the form of forceps which can be operated from the handle of the surgical instrument 1. On the body portion 3 there can be seen a pattern generating member 5 which emits light in an angle front of the surgical instrument 1, the angle is shown with a dotted line. The pattern generating member 5 has a plurality of light sources 6 which emits rays of light that generates a dotted light pattern.

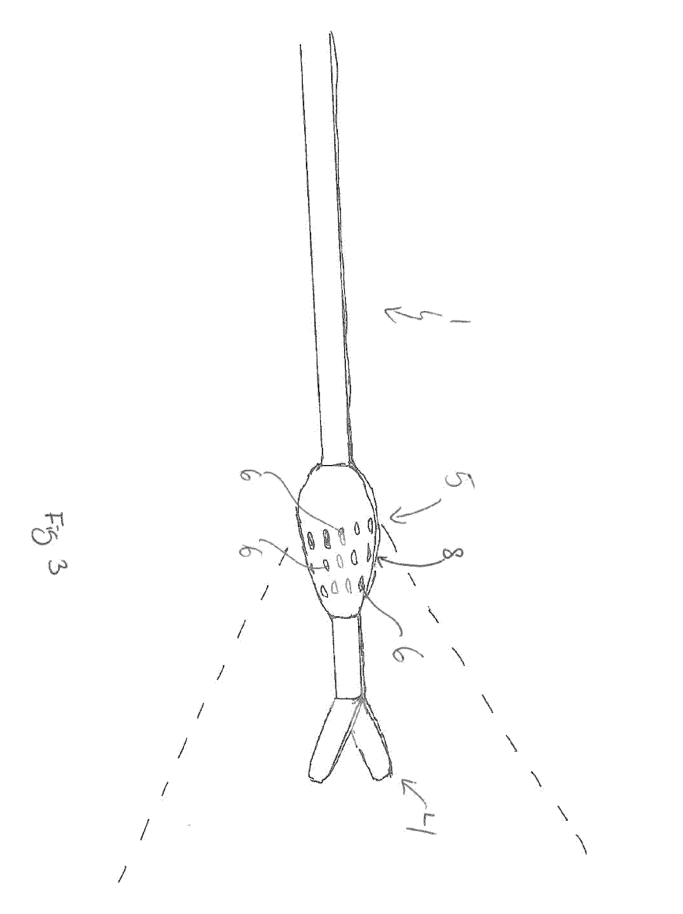

Fig. 3 shows an alternative embodiment of a pattern generating member 6. The pattern generating member 5 has a cylindrical geometry and a plurality of light sources 6 which generates light rays which forms the light pattern. The light sources 6 situated in rows and are evenly distributed on the pattern generating member 5. The rows have a plurality of light sources 6. The light sources 6 is angled such that the light sources closest to the surgical tool 4 projects its light at a smaller angle than the light sources 6 further away from the surgical tool 4. In this way the light pattern can cover a larger area.

The light source may be constructed so that no light is projected in the direction of the laparoscope in order to prevent light hitting it and thus disturb the view. This can for example be done by turning off the light in the rows which faces the endoscope. It can also be done by constructing the surgical instrument so that there are no lights facing the laparoscope or having the possibility to turn off, at least the light sources 6 which projects light on the endoscope.

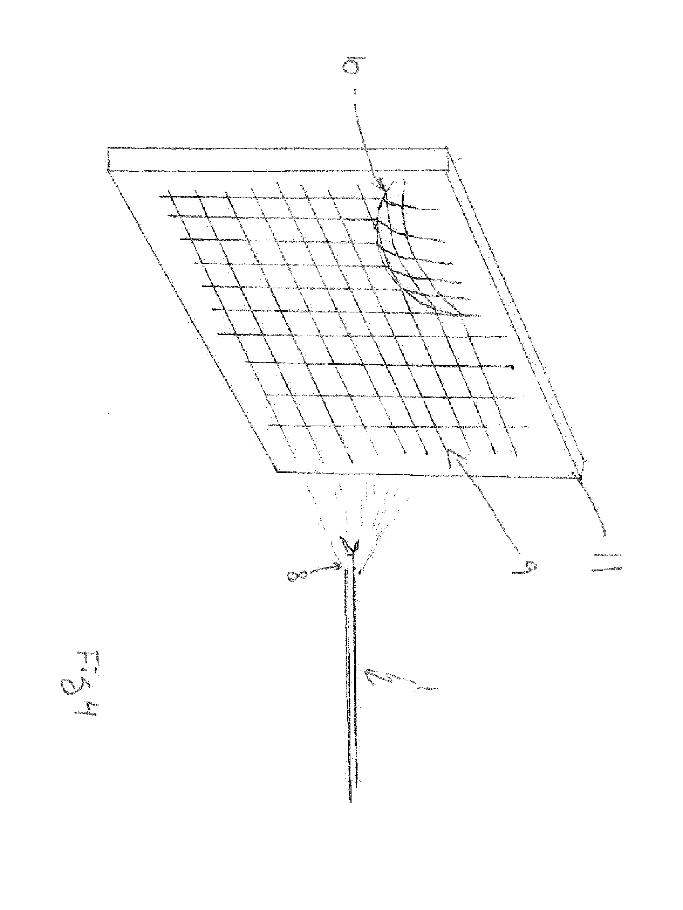

Fig. 4 is a drawing of a light pattern 9 generated by an embodiment of a surgical instrument according to the invention, the surgical instrument 1 is only shown schematic. The light pattern 9 is a grid which for illustrative purposes is shown as projected on a board 11 with a bulge 10 in the upper left corner. It can be seen that the grid makes the bulge highly visible as the contours of it stands out. Thus, a surgical instrument 1 with a pattern generating member 5 which projects a light pattern on a surgical field can be used to enhance out the three dimensional contours of the surgical field. The operator can in other words deduce the three dimensional contours of the surgical field based on a two dimensional vision of the surgical field.

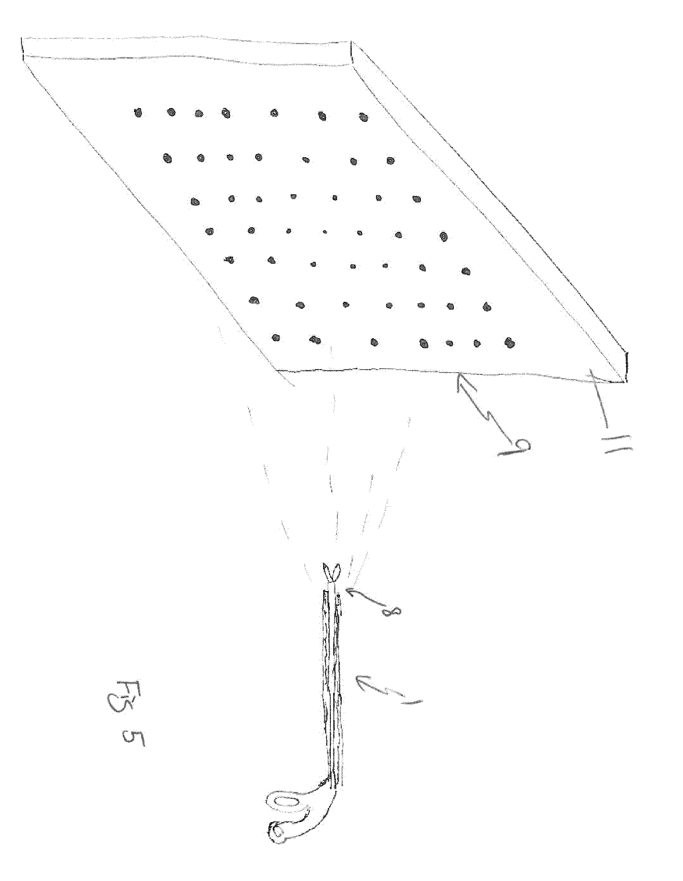

Fig. 5 shows a schematic view of a light pattern 9 on a board 11, generated by a surgical instrument 1. The light pattern 10 comprises a plurality of dots which are projected from the pattern generating member 5 on the surgical instrument 1. In similar fashion as the light pattern shown in fig. 4 it is possible for the operator to interpret variations in the light pattern as variation in the surface whereon it is projected.

The light pattern 9 (as exemplified in fig. 4 and 5) will change shape depending on the position of the surgical instrument. For example, in fig. 4 the lines will become non-parallel if the surgical instrument 1 is turned. In addition, the distance between the lines will get smaller if the surgical instrument is moved closer to the board 11 and get larger if the surgical instrument is moved away from the board 11. Thus, it is possible for the operator to deduce the position and orientation of the surgical instrument 1 in relation a surface whereon a light pattern is projected based on the geometry light pattern 9.

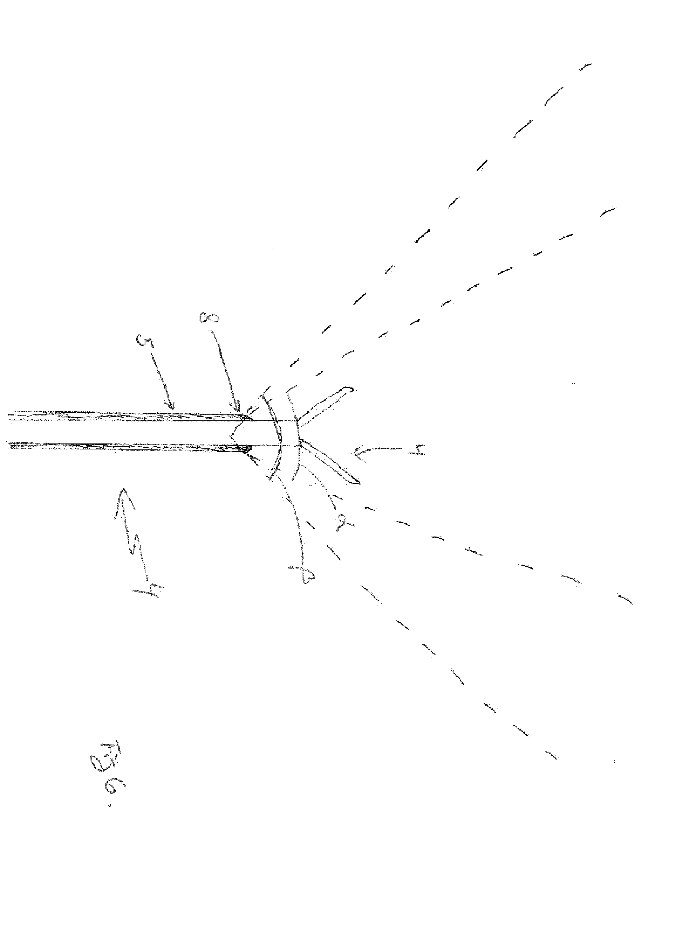

Fig. 6 shows a body portion 3 of an embodiment of a surgical instrument 1, with a surgical tool 4 and a pattern generating member 5. The figure illustrate that the pattern generating member 5, in one embodiment, can project the light pattern in an angle between 60° to 110°. The angle α is 60° and the angle β is 110°. The light pattern is projected in front of the surgical instrument 1 and can be of a cylindrical geometry such that the projected light pattern will cover an circular area right in front of the instrument 1. It is to be understood that the angles for the light pattern can be any angle and will depend on the specific use of the surgical instrument.

### Reference list:

1 surgical instrument
2 handle portion
3 body portion
4 surgical tool
5 pattern generating member
6 light sources
7 optical fibers
8 light emitting portion
9 light pattern
10 bulge
11 board

## Claims

1. Surgical instrument for use in minimal invasive surgery wherein a surgeon performs a surgical procedure within a body cavity with indirect vision of a surgical field, the surgical instrument comprises, a handle portion for manipulation of the instrument, and a body portion extending from the handle portion and comprising a surgical tool, wherein the body portion is adapted to be inserted through an incision in a body into the body cavity,
**characterised in that** the body portion further comprises a pattern generating member which, when the body part is inserted into the body cavity, projects a light pattern on an area of the surgical field such that the contours of the surgical field and the position of the instrument can be deduced by the surgeon based on indirect vision of the light pattern.

2. Surgical instrument according to claim 1, wherein the pattern generating member is detachably attached to the body portion.

3. Surgical instrument according to any of the preceding claims, wherein the light pattern is a grid.

4. Surgical instrument according to any of the preceding claims, wherein the light pattern comprises a plurality of light dots.

5. Surgical instrument according to any of the preceding claims, wherein the light pattern is generated by at least one laser and/or LED.

6. Surgical instrument according to any of the preceding claims, the projected light pattern is adapted to be able to change form, shape, position and/or colour.

7. Surgical instrument according to any of the preceding claims, wherein data on position and/or changes in the light pattern is obtained and a system is adapted to receive the data and determine the position of the surgical tool, preferably the information of the position of the surgical tool is presented to an operator.

8. Surgical instrument according to any of the preceding claims, wherein data of the wavelength of the reflected light of the light pattern is obtained and a system is adapted to receive the data and determine properties of tissue in the surgical field.

9. Surgical instrument according to any of the preceding claims, wherein the pattern generating member projects the light pattern on an area in front of the body portion covering 90 degree to the sides, preferably 60 degree.

10. Surgical instrument according to any of the preceding claims, wherein at least one light source and at least one optical waveguide is connected to the pattern generating member, wherein the at least one optical waveguide guides the light generated by the light source to where the light is projected.

11. Surgical instrument according to claim 10, wherein the light source is not intended to be inserted into the body cavity and the optical waveguide is adapted to guide the light through the incision in the body.

12. Method for training the handling of a surgical instrument having a handle portion for manipulation of the instrument, and a body portion extending from the handle portion and comprising a surgical tool, wherein the body portion further comprises a pattern generating member which projects a light pattern, the method comprises the steps;
- provide a cavity,
- provide a system for indirect vision of an area of the cavity,
- insert the surgical tool and at least a section of the body portion into the cavity,
- project the light pattern on a wall of the cavity visible by the system for indirect vision,
- move the instrument and observe, via the system for indirect vision, the changes in the light pattern.

13. Method according to claim 12, wherein data of the position and/or changes in the light pattern is obtained and forwarded to a system, and wherein the system
is adapted to determine the abilities of an operator of the surgical instruments based on an evaluation of the data of the position and/or changes in the light pattern.

14. Method for performing laparoscopic surgery using a surgical instrument according to any of the claims 1 to 11.
